# EUROPEAN PATENT APPLICATION

(11) **EP 4 534 665 A1**
(43) Date of publication of application: **09.04.2025**
(21) Application number: 23816166.5
(22) Date of filing: 02.06.2023
(51) Int. Cl.: C12N 15/09, A01H 1/00, A01K 67/027, C07K 14/195, C12N 1/15, C12N 1/19, C12N 1/21, C12N 5/10, C12N 15/11, C12N 15/63

(54) **COMPOSITION FOR INCREASING EFFICIENCY OF GENOME EDITING AND USE THEREOF**

(30) Priority: 02.06.2022 JP 2022090507
(71) Applicant: National Institute of Advanced Industrial Science and Technology, Chiyoda-ku Tokyo 100-8921 (JP)
(72) Inventor: MASEDA Hideaki, Tsukuba-shi Ibaraki 305-8560 (JP)
(74) Representative: Wasner, Marita
(86) International application number: PCT/JP2023/020607
(87) International publication number: WO 2023/234409

(57) **Abstract**

The present invention provides: a composition which is for increasing the efficiency of genome editing with a single-strand-form editing polynucleotide that is capable of modifying a target site in a double-stranded genome DNA, the composition containing at least one protein selected from the group consisting of Hfq, DnaK, and variants thereof, or a nucleic acid comprising a base sequence that encodes the protein; a genome editing kit comprising the composition and an editing polynucleotide; a method for modifying a target site in a double-stranded genome DNA of a cell or a living organism, the method comprising a step of using the genome editing kit to treat a cell or living organism; and a method comprising said step, for producing a cell or living organism in which a target site in a double-stranded genome DNA has been modified.

## Description

### Field

The present invention relates to a composition that can increase editing efficiency in genome editing using a single-stranded polynucleotide, a kit for genome editing containing the composition, and a method for genome editing using them.

### Background

Genome editing technology is a technology for modifying genomic DNA nucleotide sequences by using site-specific nucleases that can recognize and cleave specific nucleotide sequences. In particular, the genome editing technology called the CRISPR/Cas system has attracted attention as an epoch-making method that overcomes the disadvantages of early genome editing methods using ZFN (Zinc-Finger Nuclease) or TALEN (Transcription activator effector-like nuclease), such as difficulty in design, low editing efficiency, and complicated work. The CRISPR/Cas system has been used not only in academic research but also in various fields such as agriculture, forestry, fisheries, livestock breeding, and medical treatment.

The CRISPR/Cas system, however, has a problem of delivery of large site-specific nucleases into cells, as well as a problem of off-target mutations that occur at a different location from the site to be edited, which have been pointed out as issues for practical application.

The present inventor has developed a new genome editing technology using a single-stranded polynucleotide that does not require the delivery of site-specific nucleases (e.g., Patent Literatures 1 and 2). This technology has the advantage that genome editing can be performed by simply expressing or introducing the single-stranded polynucleotide into the cell, without needing to introduce components other than the single-stranded polynucleotide, typically a site-directed nuclease, into the cell.

### Citation List

### Patent Literature

Patent Literature 1: Japanese Patent No. 6899564.
Patent Literature 2: Japanese Patent Application Publication No. 2016-220639.

### Summary

### Problem to be solved

The present invention provides a new means for increasing genome editing efficiency that can be used in genome editing technologies utilizing a single-stranded polynucleotide as described in Patent Literature 1.

### Solution to Problem

The present inventor has found that the efficiency of genome editing using a single-stranded polynucleotide can be increased in the presence of a specific factor.

The present disclosure provides the followings.
[Item 1A] A composition for increasing genome-editing efficiency by a single-stranded genome-editing polynucleotide capable of modifying a target site on a double-stranded genomic DNA, containing at least one protein selected from the group consisting of Hfq, DnaK and their variants, or a nucleic acid containing a nucleotide sequence encoding the protein,
   wherein the target site consists of a primary editing site set on one genomic DNA strand and a secondary editing site on the other genomic DNA strand at a position corresponding to the primary editing site,
   the genome-editing polynucleotide consists of, in order from its 5' terminal side,
      a) a portion capable of hybridizing to the region adjacent to the 3' terminal side of the primary editing site,
         x) an optional portion consisting of a nucleotide sequence that is not identical to the nucleotide sequence of the secondary editing site, and
      b) a portion capable of hybridizing to the region adjacent to the 5' terminal side of the primary editing site, or
   the genome-editing polynucleotide consists of, in order from its 5' terminal side, portion a) and portion b), and
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
[Item 1B] A composition for increasing genome-editing efficiency by a single-stranded genome-editing polynucleotide capable of modifying a target site on a double-stranded genomic DNA, containing at least one protein selected from the group consisting of Hfq, DnaK and their variants, or a nucleic acid containing a nucleotide sequence encoding the protein,
   wherein the target site consists of a primary editing site set on one genomic DNA strand and a secondary editing site on the other genomic DNA strand at a position corresponding to the primary editing site,
   the genome-editing polynucleotide consists of, in order from its 5' terminal side,
      a) a portion consisting of a nucleotide sequence having 90% or more identity with the nucleotide sequence of the region adjacent to the 5' terminal side of the secondary editing site,
         x) an optional portion consisting of a nucleotide sequence that is not identical to the nucleotide sequence of the secondary editing site, and
      b) a portion consisting of a nucleotide sequence having 90% or more identity with the nucleotide sequence of the region adjacent to the 3' terminal side of the secondary editing site, or
   the genome-editing polynucleotide consists of, in order from its 5' terminal side, portion a) and portion b), and
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
[Item 1C] A composition for increasing genome-editing efficiency by a single-stranded genome-editing polynucleotide capable of modifying a target site on a double-stranded genomic DNA, containing at least one protein selected from the group consisting of Hfq, DnaK and their variants, or a nucleic acid containing a nucleotide sequence encoding the protein,
   wherein the target site consists of a primary editing site set on one genomic DNA strand and a secondary editing site on the other genomic DNA strand at a position corresponding to the primary editing site,
   the genome-editing polynucleotide consists of, in order from its 5' terminal side,
      a) a portion consisting of a nucleotide sequence identical to the nucleotide sequence of the region adjacent to the 5' terminal side of the secondary editing site,
         x) an optional portion consisting of a nucleotide sequence that is not identical to the nucleotide sequence of the secondary editing site, and
      b) a portion consisting of a nucleotide sequence identical to the nucleotide sequence of the region adjacent to the 3' terminal side of the secondary editing site, or
   the genome-editing polynucleotide consists of, in order from its 5' terminal side, portion a) and portion b), and
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
[Item 2A] The composition according to item 1A, 1B, or 1C, wherein Hfq is derived from bacteria.
[Item 2B] The composition according to item 1A, 1B, 1C, or 2A, wherein Hfq is derived from Escherichia coli or Pseudomonas aeruginosa.
[Item 2C] The composition according to item 1A, 1B, 1C, 2A, or 2B, wherein Hfq consists of the amino acid sequence shown in SEQ ID NO: 2 or 4.
[Item 3A] The composition according to item 1A, 1B, 1C, 2A, 2B, or 2C, wherein DnaK is derived from bacteria.
[Item 3B] The composition according to item 1A, 1B, 1C, 2A, 2B, 2C, or 3A, wherein DnaK is derived from Escherichia coli or Pseudomonas aeruginosa.
[Item 3C] The composition according to item 1A, 1B, 1C, 2A, 2B, 2C, 3A, or 3B, wherein DnaK consists of the amino acid sequence shown in SEQ ID NO: 6 or 8.
[Item 4] The composition according to item 1A, 1B, 1C, 2A, 2B, 2C, 3A, 3B, or 3C, wherein the protein has a nuclear localization signal added thereto.
[Item 5] The composition according to item 1A, 1B, 1C, 2A, 2B, 2C, 3A, 3B, 3C, or 4, wherein the protein has a nuclear localization signal consisting of the amino acid sequence shown in any one of SEQ ID NOs: 9 to 16 added thereto.
[Item. 6A] A kit for genome editing, containing:
   the composition according to item 1A, 1B, 1C, 2A, 2B, 2C, 3A, 3B, 3C, 4, or 5, and
   a single-stranded genome-editing polynucleotide, an expression vector thereof, or a conjugate of the genome-editing polynucleotide or the expression vector thereof with one or more other substances,
   wherein the genome-editing polynucleotide consists of, in order from its 5' terminal side,
      a) a portion capable of hybridizing to the region adjacent to the 3' terminal side of the primary editing site,
         x) an optional portion consisting of a nucleotide sequence that is not identical to the nucleotide sequence of the secondary editing site, and
      b) a portion capable of hybridizing to the region adjacent to the 5' terminal side of the primary editing site, or
   the genome-editing polynucleotide consists of, in order from its 5' terminal side, portion a) and portion b), and
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
[Item. 6B] A kit for genome editing, containing:
   the composition according to item 1A, 1B, 1C, 2A, 2B, 2C, 3A, 3B, 3C, 4, or 5, and
   a single-stranded genome-editing polynucleotide, an expression vector thereof, or a conjugate of the genome-editing polynucleotide or the expression vector thereof with one or more other substances,
   wherein the genome-editing polynucleotide consists of, in order from its 5' terminal side,
      a) a portion consisting of a nucleotide sequence having 90% or more identity with the nucleotide sequence of the region adjacent to the 5' terminal side of the secondary editing site,
         x) an optional portion consisting of a nucleotide sequence that is not identical to the nucleotide sequence of the secondary editing site, and
      b) a portion consisting of a nucleotide sequence having 90% or more identity with the nucleotide sequence of the region adjacent to the 3' terminal side of the secondary editing site, or
   the genome-editing polynucleotide consists of, in order from its 5' terminal side, portion a) and portion b), and
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
[Item. 6C] A kit for genome editing, containing:
   the composition according to item 1A, 1B, 1C, 2A, 2B, 2C, 3A, 3B, 3C, 4, or 5, and
   a single-stranded genome-editing polynucleotide, an expression vector thereof, or a conjugate of the genome-editing polynucleotide or the expression vector thereof with one or more other substances,
   wherein the genome-editing polynucleotide consists of, in order from its 5' terminal side,
      a) a portion consisting of a nucleotide sequence identical to the nucleotide sequence of the region adjacent to the 5' terminal side of the secondary editing site,
         x) an optional portion consisting of a nucleotide sequence that is not identical to the nucleotide sequence of the secondary editing site, and
      b) a portion consisting of a nucleotide sequence identical to the nucleotide sequence of the region adjacent to the 3' terminal side of the secondary editing site, or
   the genome-editing polynucleotide consists of, in order from its 5' terminal side, portion a) and portion b), and
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
[Item. 6D] The kit for genome editing according to item 6A, 6B, or 6C, wherein the lengths of portions a) and b) are each independently 10 to 100 nucleotides.
[Item. 7] The kit for genome editing according to item 6A, 6B, 6C, or 6D, containing the single-stranded genome-editing polynucleotide containing DNA high-affinity nucleotide analogs as constituent units, or a conjugate of the genome-editing polynucleotide with one or more other substances.
[Item 8] A method for modifying a target site on a double-stranded genomic DNA of a cell or an organism, including the step of treating the cell or the organism using the kit for genome editing according to item 6A, 6B, 6C, 6D, or 7.
[Item 9] A method for producing a cell or an organism in which a target site on a double-stranded genomic DNA has been modified, including the step of treating the cell or the organism using the kit for genome editing according to item 6A, 6B, 6C, 6D, or 7.

### Advantageous Effects of Invention

According to the present invention, genome editing can be performed efficiently.

### Brief Description of Drawings

[FIG.1] Fig. 1 is a diagram illustrating the relationship between a genome-editing polynucleotide (70EGFPdelnonPOD, SEQ ID NO: 17) used for genome editing and DNA to be edited (loss-of-function GFP gene, SEQ ID NO: 18). In the figure, only a partial sequence of the DNA to be edited is shown (the nucleotide sequence of the partial sequence sense strand is SEQ ID NO: 25, and the nucleotide sequence of the partial sequence antisense strand is SEQ ID NO: 26).
[FIG.2] Fig. 2 is a graph illustrating the genome-editing efficiency (nucleotide deletion rates) by the genome-editing polynucleotide in the presence of Hfq or DnaK in HEK293T cells.
[FIG.3] Fig. 3 is a schematic diagram illustrating the configuration of a cassette linking the mexT gene of Pseudomonas aeruginosa, chloramphenicol resistance gene (CmR), and EGFP gene, which is included in an assay plasmid for measuring genome-editing efficiency.
[FIG.4] Fig. 4 is a graph illustrating the nucleotide deletion rates in the presence of a DnaK expression vector, an expression vector for DnaK and Hfq, or an Hfq expression vector in Pseudomonas aeruginosa.
[FIG. 5] Fig. 5 is a diagram illustrating the mechanism of genome editing (substitution) by a genome-editing polynucleotide.

### Detailed Description of Invention

The following description may be based on representative embodiments or specific examples, but the present invention is not limited to such embodiments or specific examples. The upper limit value and the lower limit value of each numerical range exemplified in the present specification can be combined as desired. In the present specification, a numerical range represented using "to" or "-" means a range including numerical values at both ends thereof as an upper limit value and a lower limit value, unless otherwise noted.

In the present disclosure, genomic DNA refers to DNA contained as a genome in the cell of any living organism. Genomic DNA can be prokaryotic genomic DNA or eukaryotic genomic DNA, which may contain exogenous DNA, such as from a virus, as part of it.

A prokaryote includes, for example, Gram-negative bacteria (Pseudomonas aeruginosa, Escherichia coli, Serratia spp., Sphingomonas spp., Brucella spp., Neisseria gonorrhoeae, Burkholderia spp., Shigella spp., Salmonella spp., Acinetobacter spp., Vibrio cholerae, Klebsiella pneumoniae, Legionella spp., Helicobacter pylori, Campylobacter spp., etc.), and Gram-positive bacteria (Mycobacterium tuberculosis, Mycoplasma spp., Staphylococcus aureus, Actinomyces, Bacillus subtilis, Bacillus anthracis, etc.). A prokaryote can preferably be a Gram-negative bacterium, more preferably Pseudomonas aeruginosa, Escherichia coli, Serratia spp. or Sphingomonas spp, further more preferably Pseudomonas aeruginosa or Escherichia coli.

An example of a eukaryote is an animal, including mammals such as humans and non-human mammals (monkeys, dogs, cats, rabbits, mice, rats, guinea pigs, hamsters, cattle, pigs, goats, sheep, horses, llamas, camels, etc.); birds such as chickens; reptiles such as turtles and crocodiles; amphibians such as African clawed frogs; fish such as zebrafish, medaka, and pufferfish; chordates such as sea squirts; and arthropods such as Drosophila and silkworms.

Another example of a eukaryote is a plant, including food crops such as rice, corn, potatoes, beans, and grains, such as barley and wheat; horticultural crops (vegetables, fruit trees, and flowers), such as leafy vegetables (cabbage, asparagus, etc.), fruit vegetables (eggplant, tomato, cucumber, etc.), root vegetables (radish, carrot, etc.), other vegetables, fruits (e.g., pome fruits such as apples and pears, stone fruits such as Japanese apricots, apricots, plums, peaches, and cherries, nuts such as almonds, walnuts, and chestnuts, and citrus fruits such as tangerines and lemons, and tropical fruit trees such as tropical fruits); and ornamental plants.

Another example of a eukaryote is fungi, including yeast, molds, and basidiomycetes, such as yeast, red bread mold, matsutake mushrooms, shiitake mushrooms, enoki mushrooms, shimeji mushrooms, nameko mushrooms, and eryngii mushrooms. A eukaryote can also be a microalgae.

The sense strand of genomic DNA means to the DNA strand among DNA double strands, that is not the template for transcription within a region that is transcribed into RNA, such as a coding region. The antisense strand means to the complementary strand of the sense strand, which is the template for transcription. The coding region is not limited to a protein-coding region, and also includes a region that codes RNA that does not code a protein (e.g., miRNA, piRNA, tRNA, rRNA, snRNA, gRNA, SRP RNA, pRNA, tncRNA, sbRNA, snIRNA, SLRNA, etc.)

The terms "genome editing", "editing of genomic DNA" and "modification of genomic DNA" can be used interchangeably.

The term "polynucleotide" refers to a polymer or oligomer formed by multiple deoxyribonucleotides or ribonucleotides linked via phosphodiester bonds and can be used interchangeably with the term "nucleic acid". An example of a polynucleotide includes deoxyribonucleic acid (DNA), ribonucleic acid (RNA), and a chimeric nucleic acid containing both deoxyribonucleotide and ribonucleotide as its constituent units. A polynucleotide can contain a nucleotide analog as its constituent unit.

The terms " portion" and "region" with respect to a polynucleotide can be used interchangeably and both mean a one nucleotide or multiple contiguous nucleotides contained in the polynucleotide. The terms "portion" and "region" with respect to double-stranded genomic DNA can be used interchangeably and both mean a one nucleotide pair or multiple contiguous nucleotide pairs contained in the double-stranded genomic DNA.

In the present disclosure, a portion or region "at a position corresponding to" a portion or region of a polynucleotide refers to a portion or region complementary to the portion or region of that polynucleotide on a different polynucleotide.

Nucleotide sequence identity means to the percentage of identical nucleotides among all overlapping nucleotides in the optimal alignment calculated using algorithms known in the technical field (preferably, the algorithm can consider the gap introduction into one or both sequences for optimal alignment). Identity can be calculated, for example, by aligning two nucleotide sequences using NCBI BLAST-2 (National Center for Biotechnology Information Basic Local Alignment Search Tool-2) with default settings. When a sequence to be aligned contains uracil, the identity calculation is performed using the sequence where uracil has been replaced with thymine. When a sequence to be aligned contains a modified nucleotide or nucleotide analog, the identity calculation is performed using the sequence where the modified nucleotide or nucleotide analog has been replaced with its corresponding natural deoxyribonucleotide, i.e. adenine, thymine, guanine or cytosine.

In the present disclosure, a polynucleotide or a portion or region thereof that is " capable of hybridizing" to another polynucleotide or a portion or region thereof means that one polynucleotide or a portion or region thereof has the ability to bind to the other polynucleotide or a portion or region thereof through hydrogen bonds between complementary bases. The capability to hybridize can be confirmed by maintaining the hybridized state under a stringent condition. As taught in Berger and Kimmel (1987, Guide to Molecular Cloning Techniques Methods in Enzymology, Vol. 152, Academic Press, San Diego CA), a stringent condition can be determined based on the melting temperature (Tm) of a nucleic acid to which a probe is to be bound. When the hybridization state between two polynucleotides or their portions or regions is maintained by washing under a stringent condition, e.g., with 1 x SSC, 0.1% SDS, at 37°C, they are capable of hybridizing to each other. A stringent condition may be washing with 0.5 x SSC, 0.1 % SDS, at 42°C, or with 0.1 x SSC, 0.1% SDS, at 65°C.

The term "expression vector" as used with respect to a polynucleotide refers to a vector containing a polynucleotide and equipped with a mechanism to express the polynucleotide in the cell into which the vector is introduced. A polynucleotide expression vector can also be represented as a vector capable of expressing the polynucleotide in a cell into which the vector is introduced. An example of a polynucleotide expression vector is a vector containing the polynucleotide operably linked to a promoter or other regulatory sequence.

The term "operably linked" as used with respect to a polynucleotide means that a regulatory sequence, such as a promoter, is located sufficiently close to the polynucleotide such that it can influence the expression of the polynucleotide. For example, "a polynucleotide operably linked to a promoter" means that the polynucleotide is linked such that it is expressed under the control of the promoter.

Amino acid sequence identity means the percentage of identical amino acids among all overlapping amino acids in the optimal alignment calculated using algorithms known in the art (preferably, the algorithm can consider the gap introduction into one or both sequences for optimal alignment). The identity can be calculated, for example, by aligning two amino acid sequences using NCBI BLAST-2 (National Center for Biotechnology Information Basic Local Alignment Search Tool-2) with default settings. When a sequence to be aligned contains a modified amino acid or amino acid analog, the identity calculation is performed using the sequence where the modified amino acid or amino acid analog has been replaced with its corresponding natural amino acid.

### Promoting composition for genome editing

The present disclosure provides, in one aspect, a composition for increasing genome-editing efficiency by a single-stranded genome-editing polynucleotide capable of modifying a target site on a double-stranded genomic DNA (also referred to as a "promoting composition for genome editing" in the present specification), containing at least one protein selected from the group consisting of Hfq, DnaK and their variants, or a nucleic acid containing or consisting of a nucleotide sequence encoding the protein, wherein the genome-editing polynucleotide consists of, in order from its 5' terminal side,
a) a portion capable of hybridizing to the region adjacent to the 3' terminal side of the primary editing site,
   x) an optional portion consisting of a nucleotide sequence that is not identical to the nucleotide sequence of the secondary editing site, and
b) a portion capable of hybridizing to the region adjacent to the 5' terminal side of the primary editing site, or
consists of, in order from its 5' terminal side, portion a) and portion b).

In an embodiment, the promoting composition for genome editing contains at least one protein selected from the group consisting of Hfq, DnaK and their variants.

Hfq (host factor Qβ phage) is a protein belonging to the Sm-like protein family, which is an RNA-binding motif, and is widely found in bacteria. Hfq functions as an RNA chaperone that binds to both small RNAs (sRNAs), which are post-transcriptional regulators of mRNA expression, and their target mRNAs, promoting base pair formation between them.

In the present disclosure, Hfq derived from bacteria or archaea can be used. Examples of Hfq include Hfq from bacteria belonging to Proteobacteria phylum, for example, Escherichia coli Hfq (HFQ_ECOLI), Shigella flexneri Hfq (HFQ_SHIFL), Salmonella typhimurium Hfq (HFQ_SALTY), Yersinia enterocolitica Hfq (HFQ_YEREN), Yersinia pestis Hfq (HFQ_YERPE), Erwinia carotovora Hfq (HFQ_ERWCA), Haemophilus influenzae Hfq (HFQ_HAEIN), Pasteurella multocida Hfq (HFQ_PASMU), Vibrio cholerae Hfq (HFQ_VIBCH), Pseudomonas aeruginosa Hfq (HFQ_PSEAE), Xanthomonas axonopodis Hfq (HFQ_XANAC), Xanthomonas campestris Hfq (HFQ_XANCP), Xylella fastidiosa Hfq (HFQ_XYLFA), Neisseria meningitidis Hfq (HFQ_NEIMA), Ralstonia solanacearum Hfq (HFQ_RALSO), Agrobacterium tumefaciens Hfq (HFQ_AGRT5), Brucella melitensis Hfq (HFQ_BRUME), Rhizobium loti Hfq (HFQ_RHILO), Azorhizobium caulinodans Hfq (HFQ_AZOCA), Caulobacter crescentus Hfq (HFQ_CAUCR); Hfq from bacteria belonging to Aquificae phylum, for example, Aquifex aeolicus Hfq (HFQ_AQUAE); Hfq from bacteria belonging to Thermotogae phylum, for example, Thermotoga maritima Hfq (HFQ_THEMA); Hfq from bacteria belonging to Firmicutes phylum, for example, Clostridium acetobutylicum Hfq (HFQ_CLOAB), Clostridium perfringens Hfq (HFQ_CLOPE), Bacillus halodurans Hfq (HFQ_BACHD), Bacillus subtilis Hfq (HFQ_BACSU), Thermoanaerobacter tengcongensis Hfq (HFQ_THETN), S. aureus Hfq (Q99UG9); and Hfq from archaea, for example, Methanocaldococcus jannaschii Hfq (Q58830).

DnaK is a heat shock protein belonging to the HSP (Heat shock protein) 70 family proteins. HSP70 family proteins are highly conserved in all organisms. DnaK functions as a molecular chaperone that controls protein folding by binding to synthesized proteins.

In the present disclosure, DnaK derived from bacteria can be used. Examples of DnaK include DnaK from bacteria belonging to Proteobacteria phylum, for example, Escherichia coli DnaK (DNAK_ECOLI), Salmonella enteritidis DnaK (DNAK_SALEN), Pseudomonas aeruginosa DnaK (DNAK_PSEAE), Pseudomonas fluorescence DnaK (DNAK_PSEFL), Ralstonia solanacaearum DnaK (DNAK_RALSO), Paraburkholderia fungorum DnaK (DNAK_9BURK); DnaK from bacteria belonging to Acidobacteriota phylum, for example, Acidobacteria bacterium DnaK (DNAK_9BACT); and DnaK from bacteria belonging to Planctomycetes phylum, for example, Planctomycetes bacterium DnaK (DNAK_PLABA).

The amino acid sequences of Hfq and DnaK are described in known databases. For example, in the Reference Sequence Database of the National Center for Biotechnology Information (NCBI), the amino acid sequence of E. coli Hfq (SEQ ID NO: 2; nucleotide sequence of coding DNA shown in SEQ ID NO: 1) is registered as NP_418593.1, and the amino acid sequence of E. coli DnaK (SEQ ID NO: 6; nucleotide sequence of coding DNA shown in SEQ ID NO: 5) is registered as NP_414555.1. The amino acid sequence of P. aeruginosa Hfq (SEQ ID NO: 4; nucleotide sequence of coding DNA shown in SEQ ID NO: 3) is registered as NP_253631.1, and the amino acid sequence of P. aeruginosa DnaK (SEQ ID NO: 8; nucleotide sequence of coding DNA shown in SEQ ID NO: 7) is registered as NP_253449.1.

A variant of Hfq is a protein that contains or consists of an amino acid sequence in which one or more amino acids are deleted or substituted in the amino acid sequence of Hfq, or contains or consists of an amino acid sequence in which one or more amino acids are inserted into the amino acid sequence of Hfq, and has an activity of increasing genome-editing efficiency (hereinafter also referred to as "editing-promoting ability") by a single-stranded genome-editing polynucleotide. Similarly, a variant of DnaK is a protein that contains or consists of an amino acid sequence in which one or more amino acids are deleted or substituted in the amino acid sequence of DnaK, or contains or consists of an amino acid sequence in which one or more amino acids are inserted into the amino acid sequence of DnaK, and has editing-promoting ability.

An example of these variants is a protein consisting of an amino acid sequence having at least 70% or more, preferably 80% or more, more preferably 90% or more, further more preferably 95% or more, and especially preferably 97%, 98% or 99% or more identity with the amino acid sequence of Hfq or DnaK, and having editing-promoting ability. Also, a polypeptide fragment of Hfq or DnaK that has editing-promoting ability is an example of the variants.

It is preferable that amino acid substitutions that give rise to variants are so-called conservative substitutions. Examples of conservative substitutions include substitutions between amino acids such as glycine (Gly) and proline (Pro), glycine and alanine (Ala) or valine (Val), leucine (Leu) and isoleucine (Ile), glutamic acid (Glu) and glutamine (Gln), aspartic acid (Asp) and asparagine (Asn), cysteine (Cys) and threonine (Thr), threonine and serine (Ser) or alanine, and lysine (Lys) and arginine (Arg).

When genome-editing efficiency by a genome-editing polynucleotide is increased by coexistence with a variant, the variant can be confirmed to have editing-promoting ability.

Although not bound by theory, the editing-promoting ability of Hfq and DnaK is thought to be based on their activity as chaperones. Therefore, an example of Hfq variants and DnaK variants can be a protein consisting of an amino acid sequence having at least 70% or more, preferably 80% or more, more preferably 90% or more, further more preferably 95% or more, and especially preferably 97%, 98% or 99% or more identity with the amino acid sequence of Hfq or DnaK, and having chaperone activity.

Hfq, DnaK and their variants can be produced by genetic engineering methods in which expression vectors containing DNAs encoding these proteins are introduced into appropriate host cells, such as E. coli, insect cells, and animal cells, and the proteins are expressed therein. Genetic engineering methods including preparation of DNAs and expression vectors, types of host cells and methods for introducing expression vectors, protein expression and purification, etc. are well known to those skilled in the art and can be performed according to instructions in experimental operation manuals describing various techniques in detail.

Cell-free protein synthesis using DNA or mRNA encoding Hfq, DnaK and their variants is also one of the genetic engineering production methods. Examples of cell-free protein synthesis systems include systems using cell extracts from E. coli, wheat germ, yeast, rabbit reticulocytes, insect cells, and mammalian cultured cells, and reconstituted systems composed of factors necessary for protein synthesis.

Hfq, DnaK and their variants can also be produced by organic chemical production methods such as Fmoc method (fluorenylmethyloxycarbonyl method) or tBoc method (t-butyloxycarbonyl method).

In an embodiment, Hfq, DnaK and their variants may have a nuclear localization signal (NLS) inserted or added thereto. A nuclear localization signal, also called a nuclear localization sequence, is an amino acid sequence known as a region where several lysine residues and/or arginine residues are clustered in the primary structure of proteins that are synthesized in cells and transported to the nucleus.

Examples of the nuclear localization signal that is known and can be used in the present disclosure include PKKKRKV (SEQ ID NO: 9) in SV40 T antigen and its basic motif KKKRK (SEQ ID NO: 10), PAAKRVKLD (SEQ ID NO: 11) in c-Myc, PQPKKKP (SEQ ID NO: 12) in p53, QRKRQK (SEQ ID NO: 13) in NF-κB p50, KRPAATKKAGQAKKKK (SEQ ID NO: 14) in nucleoplasmin, MSRRRKANPTKLSENAKKLAKEVEN (SEQ ID NO: 15) in GL-13, and KLKIKRPVK (SEQ ID NO: 16) in TUS protein.

The nuclear localization signal can be inserted or added at any position as long as it does not affect the editing-promoting ability of Hfq, DnaK and their variants. In a preferred embodiment, the nuclear localization signal is added to the N-terminal side or C-terminal side of Hfq, DnaK and their variants.

Hfq, DnaK and their variants may have a further other peptide added thereto in addition to the insertion or addition of the nuclear localization signal, as long as they have editing-promoting ability. Examples of such further other peptide include the Fc region of human IgG (Fc protein), His tag, GST tag, HA tag, and FLAG tag.

Hfq, DnaK and their variants in forms having nuclear localization signal and/or the above further other peptide inserted or added thereto can be produced by the above-described genetic engineering or organic chemical production methods.

Hfq, DnaK and their variants may be chemically modified as long as they have editing-promoting ability. Examples of chemical modification include modification of an amino group of an amino acid residue (biotinylation, myristoylation, palmitoylation, acetylation, maleimidation, methylation, malonylation, etc.), modification of a carboxyl group of an amino acid residue (amidation, esterification, etc.), modification of a thiol group of an amino acid residue (farnesylation, geranylation, methylation, palmitoylation, etc.), modification of a hydroxyl group of an amino acid residue (phosphorylation, sulfation, etc.), PEGylation, and glycosylation. Chemical modification can be performed on one or more amino acid residues.

In an embodiment, the promoting composition for genome editing contains a nucleic acid containing or consisting of a nucleotide sequence encoding at least one protein selected from the group consisting of Hfq, DnaK and their variants. When Hfq, DnaK and their variants are in forms having the nuclear localization signal and/or the above further other peptide inserted or added thereto, the nucleotide sequence can be one that encodes Hfq, DnaK or their variants in forms with the nuclear localization signal and/or the above further other peptide inserted or added thereto.

The nucleic acid may be DNA or mRNA, and when it is DNA, it is preferably in the form of an expression vector. The nucleic acid may have, in one molecule, one nucleotide sequence encoding one protein selected from the group consisting of Hfq, DnaK and their variants, or may have two or more nucleotide sequences encoding two or more proteins selected from the group.

Examples of the expression vector that can be used in the present disclosure include a plasmid vector, a viral vector (e.g., a retroviral vector, a lentiviral vector, a Sendai virus vector, an adenoviral vector, an adeno-associated viral vector), and an RNA vector.

The promoting composition for genome editing is not limited as long as it is a composition containing at least one protein selected from the group consisting of Hfq, DnaK and their variants, or a nucleic acid containing or consisting of a nucleotide sequence encoding the protein, and may include, for example, liquid such as water, saline, phosphate buffered saline, cell culture medium, and agent such as transfection reagent.

### Genome-editing polynucleotide

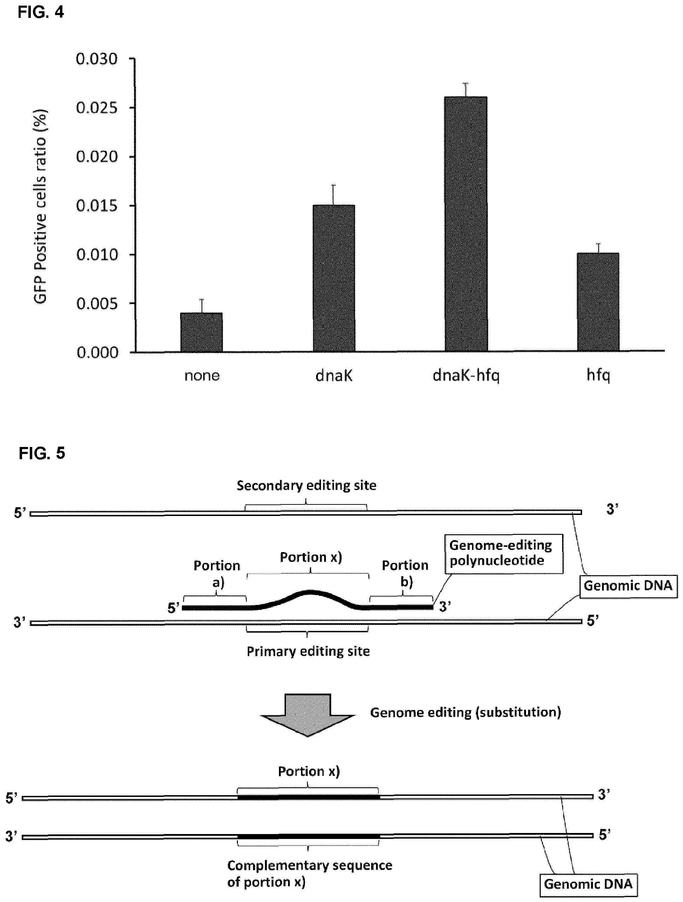

The promoting composition for genome editing can increase the efficiency of modifying a target site on a double-stranded genomic DNA by a single-stranded genome-editing polynucleotide that consists of, in order from its 5' terminal side,
a) a portion capable of hybridizing to the region adjacent to the 3' terminal side of the primary editing site,
   x) an optional portion consisting of a nucleotide sequence that is not identical to the nucleotide sequence of the secondary editing site, and
b) a portion capable of hybridizing to the region adjacent to the 5' terminal side of the primary editing site, or
consists of, in order from its 5' terminal side, portion a) and portion b). The details of the genome-editing polynucleotide are described below (see also Fig. 5).

In the present disclosure, a target site refers to one nucleotide pair or multiple contiguous nucleotide pairs, or a position between two contiguous nucleotide pairs on a double-stranded genomic DNA, that is intended to be modified. A target site consists of a primary editing site set on one genomic DNA strand and a secondary editing site on the other genomic DNA strand at a position corresponding to the primary editing site. For example, when the intended modification is to replace or delete one nucleotide pair or multiple contiguous nucleotide pairs in double-stranded genomic DNA, the primary editing site is set at one nucleotide or multiple nucleotides intended to be replaced or deleted on one genomic DNA strand. When the intended modification is to insert one nucleotide pair or multiple contiguous nucleotide pairs into double-stranded genomic DNA, the primary editing site is set between two contiguous nucleotides into which one nucleotide or multiple contiguous nucleotides intended to be inserted on one genomic DNA strand.

The primary editing site can be set at any one nucleotide or multiple contiguous nucleotides, or between any two contiguous nucleotides, on one genomic DNA strand. The primary editing site is not limited to being set at a specific one nucleotide or multiple contiguous nucleotides, or between two specific contiguous nucleotides.

When the primary editing site is set at one nucleotide or multiple contiguous nucleotides, the region adjacent to the 5' terminal side of the primary editing site and the region adjacent to the 3' terminal side of the primary editing site are separated by the length of the primary editing site on the genomic DNA. When the primary editing site is set at a position between two contiguous nucleotides, the region adjacent to the 5' terminal side of the primary editing site and the region adjacent to the 3' terminal side of the primary editing site are adjacent to each other on the genomic DNA.

When the primary editing site is set at multiple contiguous nucleotides on one genomic DNA strand, the number of contiguous nucleotides is not particularly limited, and can be, for example, 2 nucleotides or more, 3 nucleotides or more, 4 nucleotides or more, 5 nucleotides or more, 6 nucleotides or more, 7 nucleotides or more, or 8 nucleotides or more, and can be, for example, 2000 nucleotides or less, 1000 nucleotides or less, 900 nucleotides or less, 800 nucleotides or less, 700 nucleotides or less, 600 nucleotides or less, 500 nucleotides or less, 450 nucleotides or less, 400 nucleotides or less, 350 nucleotides or less, 300 nucleotides or less, 250 nucleotides or less, 200 nucleotides or less, 150 nucleotides or less, 100 nucleotides or less, 90 nucleotides or less, 80 nucleotides or less, 70 nucleotides or less, 60 nucleotides or less, 50 nucleotides or less, 40 nucleotides or less, 30 nucleotides or less, 20 nucleotides or less, 15 nucleotides or less, 12 nucleotides or less, 10 nucleotides or less, or 9 nucleotides or less.

The primary editing site may be set on either strand of the two strands constituting genomic DNA. The secondary editing site is to be set on the strand of genomic DNA on which the primary editing site is not set. For example, when modification of the region of double-stranded genomic DNA that is transcribed into RNA is intended, the primary editing site may be set on the sense strand or on the antisense strand.

Portion a) of the genome-editing polynucleotide can hybridize with the region adjacent to the 3' terminal side of the primary editing site.

Portion a) can also be a portion consisting of a nucleotide sequence having 90% or more identity with the nucleotide sequence of the region adjacent to the 5' terminal side of the secondary editing site. Here, the identity is preferably 95% or more, more preferably 97% or more, further more preferably 99% or more, even more preferably 99.5% or more, and especially preferably 99.9% or more.

In a preferred embodiment, portion a) consists of a nucleotide sequence complementary to the nucleotide sequence of the region adjacent to the 3' terminal side of the primary editing site, i.e., a nucleotide sequence identical to the nucleotide sequence of the region adjacent to the 5' terminal side of the secondary editing site.

Portion b) of the genome-editing polynucleotide can hybridize with the region adjacent to the 5' terminal side of the primary editing site.

Portion b) can also be a portion consisting of a nucleotide sequence having 90% or more identity with the nucleotide sequence of the region adjacent to the 3' terminal side of the secondary editing site. Here, the identity is preferably 95% or more, more preferably 97% or more, further more preferably 99% or more, even more preferably 99.5% or more, and especially preferably 99.9% or more.

In a preferred embodiment, portion b) consists of a nucleotide sequence complementary to the nucleotide sequence of the region adjacent to the 5' terminal side of the primary editing site, i.e., a nucleotide sequence identical to the nucleotide sequence of the region adjacent to the 3' terminal side of the secondary editing site.

The lengths of portions a) and b) are not particularly limited and can be set independently. The lengths of one of portions a) and b) can be, respectively, for example, 10 nucleotides or more, 20 nucleotides or more, 30 nucleotides or more, 35 nucleotides or more, 40 nucleotides or more, 45 nucleotides or more, 50 nucleotides or more, 60 nucleotides or more, 70 nucleotides or more, 80 nucleotides or more, 90 nucleotides or more, or 100 nucleotides or more, and can be 1000 nucleotides or less, 500 nucleotides or less, 300 nucleotides or less, 200 nucleotides or less, 190 nucleotides or less, 180 nucleotides or less, 170 nucleotides or less, 160 nucleotides or less, 150 nucleotides or less, 140 nucleotides or less, 130 nucleotides or less, 120 nucleotides or less, 110 nucleotides or less, 100 nucleotides or less, 90 nucleotides or less, 80 nucleotides or less, 70 nucleotides or less, 60 nucleotides or less, or 50 nucleotides or less.

The length of one of portions a) and b) can be, respectively, for example, 10 to 1000 nucleotides, 10 to 500 nucleotides, 10 to 300 nucleotides, 10 to 200 nucleotides, 10 to 100 nucleotides, 32 to 100 nucleotides, 35 to 70 nucleotides, or 35 to 50 nucleotides.

Portion x) is an optional portion consisting of a nucleotide sequence that is not identical to the nucleotide sequence of the secondary editing site and is determined by how the target site is to be modified. For example, when the intended modification is to replace one nucleotide pair or multiple contiguous nucleotide pairs in double-stranded genomic DNA, then portion x) consists of one nucleotide or multiple contiguous nucleotides intended to replace the secondary editing site. When the intended modification is to delete one nucleotide pair or multiple contiguous nucleotide pairs in double-stranded genomic DNA, then portion x) is not included in the genome-editing polynucleotide. When the intended modification is to insert one nucleotide pair or multiple contiguous nucleotide pairs into double-stranded genomic DNA, then portion x) consists of one nucleotide or multiple contiguous nucleotides intended to be inserted into the secondary editing site.

Portion x) consists of a nucleotide sequence that is not identical to the nucleotide sequence of the secondary editing site. Portion x) can consist of any nucleotide sequence, as long as it is not an exact match to the nucleotide sequence of the secondary editing site, and can consist of, as an example, a nucleotide sequence with less than 90% identity to the nucleotide sequence of the secondary editing site. The identity is, for example, 50% or less, preferably 40% or less, more preferably 30% or less, further more preferably 20% or less, even more preferably 10% or less, and especially preferably 5% or less.

The length of portion x) is not particularly limited, and can be, for example, 2000 nucleotides or less, 1000 nucleotides or less, 900 nucleotides or less, 800 nucleotides or less, 700 nucleotides or less, 600 nucleotides or less, 500 nucleotides or less, 400 nucleotides or less, 300 nucleotides or less, 200 nucleotides or less, 100 nucleotides or less 90 nucleotides or less, 80 nucleotides or less, 70 nucleotides or less, 60 nucleotides or less, 50 nucleotides or less, 40 nucleotides or less, 30 nucleotides or less, 20 nucleotides or less, 10 nucleotides or less, 9 nucleotides or less, 8 nucleotides or less, 7 nucleotides or less, 6 nucleotides or less, 5 nucleotides or less, 4 nucleotides or less, 3 nucleotides or less, 2 nucleotides or less, or 1 nucleotide.

The genome-editing polynucleotide consists of portions a), x) and b), or portions a) and b), in order from the 5' terminal side. Thus, the length of the genome-editing polynucleotide can be determined by summing the lengths of each of the portions consisting of the genome-editing polynucleotide, that is, by summing the lengths of portion a), portion x), and portion b), or by summing the lengths of portions a) and b).

The genome-editing polynucleotide contains one or more DNA high-affinity nucleotide analogs in one or both of portions a) and b). The original nucleotide can be replaced with a DNA high-affinity nucleotide analog so that the bases of the original nucleotide and the DNA high-affinity nucleotide analog are common, for example, when the original base is adenine, it can be replaced with a DNA high-affinity nucleotide analog containing adenine or an adenine analog. Multiple DNA high-affinity nucleotide analogs may be included adjacently, or they may be included intermittently without being adjacent. When included in both portions a) and b), the positions at which the DNA high-affinity nucleotide analogs are included can be chosen independently, and there is no requirement for the same number of them to be included in both portions.

A DNA high-affinity nucleotide analog is an artificial nucleic acid constituent unit with high binding affinity for DNA that can form polymer structures similar to natural nucleic acids. Examples of the DNA high-affinity nucleotide analog include a nucleotide with a cross-link between the oxygen atom at the 2' position and the carbon atom at the 4' position of the ribose ring, a peptide nucleic acid (PNA) with N-(2-aminoethyl)glycine instead of deoxyribose or ribose, and a morpholino nucleic acid with a morpholine ring instead of deoxyribose or ribose.

In an embodiment, the DNA high-affinity nucleotide analog is a nucleotide in which the oxygen atom at the 2' position and the carbon atom at the 4' position of the ribose ring are cross-linked, examples of which include LNA (Locked Nucleic Acid) in which the oxygen atom at the 2' position and the carbon atom at the 4' position are cross-linked through a methylene bridge, ENA in which they are cross-linked through an ethylene bridge, BNA (Bridged Nucleic Acid)^{COC} in which they are cross-linked through -CH₂OCH₂-, BNA^{NC} in which they are cross-linked through -NR-CH₂- (R is methyl or a hydrogen atom), cMOE in which they are cross-linked through -CH₂(OCH₃)-, cEt in which they are cross-linked through -CH₂(CH₃)-, AmNA in which they are cross-linked through an amide bridge, and scpBNA in which they are cross-linked through a methylene bridge to form a cyclopropane at the 6' position.

The genome-editing polynucleotide may further contain a chemically modified nucleotide in addition to the DNA high-affinity nucleotide analog as its constituent unit. Examples of the chemically modified nucleotide include a nucleotide in which the phosphate group is replaced with a chemically modified phosphate group such as phosphorothioate (PS), methylphosphonate, and phosphorodithionate, etc., a nucleotide in which the hydroxyl group at the 2' position of the sugar (ribose) portion is replaced with -OR (R represents, for example, CH₃ (2'-O-Me), CH₂CH₂OCH₃ (2'-MOE), CH₂CH₂NHC(NH)NH₂, CH₂CONHCH₃, CH₂CH₂CN, etc.), a nucleotide in which a methyl group or cationic functional group is introduced at the 5-position of the pyrimidine base, and a nucleotide in which the carbonyl group at the 2-position of the pyrimidine base is replaced with a thiocarbonyl group. Furthermore, examples include a nucleotide in which the phosphate portion or hydroxyl portion is modified with, for example, biotin, an amino group, a lower alkylamine group, an acetyl group, etc., but are not limited to these nucleotides.

The genome-editing polynucleotide containing a DNA high-affinity nucleotide analog and optionally a chemically modified nucleotide can be produced by known synthetic methods.

The genome-editing polynucleotide in the present disclosure can modify genomic DNA in a cell as desired, similar to the single-stranded polynucleotide disclosed in Patent Literature 1 (Japanese Patent No. 6899564). Unlike conventional genome-editing technologies, the genome-editing polynucleotide does not require the introduction of site-specific nucleases (ZFN proteins, TALEN proteins, Cas proteins, etc.), guide RNAs, or their expression vectors into the cell.

Although not bound by theory, portion a) of the genome-editing polynucleotide hybridizes with the region adjacent to the 3' terminal side of the primary editing site set on one genomic DNA strand, while portion b) hybridizes with the region adjacent to the 5' terminal side of the primary editing site. As a result, when the genome-editing polynucleotide consists of portions a), x), and b), the portion x) protrudes, and when the genome-editing polynucleotide consists of portions a) and b), the primary editing site on the genomic DNA protrudes, without hybridizing to other sequences. This protruding segment is recognized by a repair system, allowing for modification of the target site on the double-stranded genomic DNA.

Specifically, when it is desired to insert one nucleotide pair or multiple contiguous nucleotide pairs in double-stranded genomic DNA, the primary editing site is set between two contiguous nucleotides where insertion is desired on one genomic DNA strand; portions a) and b) are set as described above; and portion x) is set at one nucleotide or multiple contiguous nucleotides that are desired to be inserted into the secondary editing site. By performing genome editing using the genome-editing polynucleotide consisting of portions a), x), and b) designed in this manner, a portion consisting of the nucleotide sequence complementary to portion x) can be inserted into the primary editing site, and portion x) can be inserted into the secondary editing site.

If it is desired to replace one nucleotide pair or multiple contiguous nucleotide pairs in double-stranded genomic DNA, the primary editing site is set at one nucleotide or multiple contiguous nucleotides where substitution is desired on one genomic DNA strand; portions a) and b) are set as described above; and portion x) is set at one nucleotide or multiple contiguous nucleotides that are desired to replace the secondary editing site. By performing genome editing using the genome-editing polynucleotide consisting of portions a), x), and b) designed in this manner, a portion consisting of the nucleotide sequence complementary to portion x) can be inserted into the primary editing site, and portion x) can be inserted into the secondary editing site (shown in Fig. 5).

If it is desired to delete one nucleotide pair or multiple contiguous nucleotide pairs in double-stranded genomic DNA, the primary editing site is set at one nucleotide or multiple contiguous nucleotides where deletion is desired on one genomic DNA strand; and portions a) and b) are set as described above. By performing genome editing using the genome-editing polynucleotide consisting of portions a) and b) designed in this manner, the primary editing site can be deleted from one genomic DNA strand and the secondary editing site can be deleted from the other genomic DNA strand, i.e., the target site can be deleted from the double-stranded genomic DNA.

Although not bound by theory, considering that there are many transcribed RNAs in the vicinity of genomic DNA, for example, when the target site is in a genomic DNA region that is transcribed into RNA and the primary editing site is set on the genomic DNA sense strand, the genome-editing efficiency is suggested to be not good. This is because the genome-editing polynucleotide may bind, rather than to the region adjacent to the 3' terminal side of the primary editing site and the region adjacent to the 5' terminal side of the primary editing site on the genomic DNA sense strand, preferentially to the region with the same nucleotide sequence as the above two regions in the surrounding RNAs. When genome-editing efficiency is a priority, it is preferable to set the primary editing site on the antisense strand rather than on the sense strand of genomic DNA.

As described above, portion a) of the genome-editing polynucleotide can hybridize with the region adjacent to the 3' terminal side of the primary editing site set on one genomic DNA strand, and portion b) of the genome-editing polynucleotide can hybridize with the region adjacent to the 5' terminal side of the primary editing site, thereby modifying the target site on the double-stranded genomic DNA. Therefore, the genome-editing polynucleotide may have any nucleotide sequence added to one or both ends, i.e., to the 5' terminal of portion a) and/or to the 3' terminal of portion b), as long as the addition of the sequence does not affect hybridization of portion a) to the region adjacent to the 3' terminal side of the primary editing site, and portion b) to the 5' terminal side of the primary editing site. The genome-editing polynucleotide in the present disclosure encompasses those to which such an optional nucleotide sequence is added.

For the genome-editing polynucleotide with an optional nucleotide sequence added at its terminal, calculation of the identity with each of the regions adjacent to the 5' terminal side and the 3' terminal side of the secondary editing site is performed between the nucleotide sequence of portion a) and the nucleotide sequence of the region adjacent to the 5' terminal side of the secondary editing site, and between the nucleotide sequence of portion b) and the nucleotide sequence of the region adjacent to the 3' terminal side of the secondary editing site. The added optional nucleotide sequence is considered to be absent in the identity calculation.

### Kit for genome editing

The present disclosure provides, in one aspect, a kit for genome editing that contains the aforementioned genome-editing polynucleotide and the aforementioned promoting composition for genome editing. The kit for genome editing can contain, instead of the genome-editing polynucleotide, an expression vector of the genome-editing polynucleotide, or a conjugate of the genome-editing polynucleotide or the expression vector of the genome-editing polynucleotide with one or more other substances.

Other substances conjugated to the genome-editing polynucleotide are not limited as long as they can form a conjugate with the genome-editing polynucleotide and do not interfere with the function of the genome-editing polynucleotide, i.e., the function of modifying the target site on genomic DNA. Examples of other substances conjugated to the genome-editing polynucleotide include functional peptides such as nuclear transfer signals and membrane-permeable peptides; hydrophilic polymers such as polyethylene glycol and dextran; and labeling substances such as radioisotopes, fluorescein and its derivatives, rhodamine and its derivatives, fluorescent proteins, phycobiliproteins, biotin, and avidin.

The kit for genome editing can further contain a reagent including a nucleic acid transfer reagent such as a transfection reagent, and a buffer solution.

The components of the kit for genome editing, such as the aforementioned genome-editing polynucleotide, its expression vector or its conjugate, the aforementioned promoting composition for genome editing, each of the reagents, may be contained in the kit for genome editing in separate forms, or may be contained in the kit for genome editing in the form of a composition in which multiple components are combined.

The kit for genome editing may further contain, for example, an instrument to be used in genome editing, instructions for use.

In an embodiment, the kit for genome editing is a kit for genome editing for the treatment or prevention of a disease, and can contain, in addition to a therapeutically or prophylactically effective amount of the genome-editing polynucleotide, its expression vector, or a conjugate of the genome-editing polynucleotide or its expression vector with one or more other substances, other pharmaceutically acceptable components, such as a pharmaceutically acceptable additive (buffer, stabilizer, preservative, excipient, etc.), and a pharmaceutically acceptable medium (water, saline, phosphate buffered saline (PBS), etc.). The kit for genome editing for the treatment or prevention of a disease can be used to treat or prevent a disease caused by an abnormality in genomic DNA.

### Method for genome editing and method for producing genome-edited cell or organism

The present disclosure provides, in one aspect, a method for modifying a target site on double-stranded genomic DNA of a cell or an organism (also referred to as a "method for genome editing" in the present disclosure), including the step of treating the cell or the organism using the aforementioned kit for genome editing. The present disclosure also provides, in one aspect, a method for producing a cell or an organism in which a target site on double-stranded genomic DNA has been modified (also referred to as a "method for producing a genome-edited cell or organism" in the present disclosure), including the step of treating the cell or the organism using the aforementioned kit for genome editing.

The cell to be treated using the kit for genome editing is a cell whose genomic DNA is desired to be modified and can originate from a variety of organisms, including the prokaryotes and eukaryotes mentioned above. If the cell is a cell of a multicellular organism, the cell can be derived from various tissues of the organism. The cell may be a somatic cell or a germ cell. The cell may be a differentiated or undifferentiated cell, a tissue stem cell, an embryonic stem cell, or an induced pluripotent stem cell (iPS cell).

The cell to be treated using the kit for genome editing may be in the form of a single cell or a cell population containing one or more types of cells.

The organism to be treated using the kit for genome editing is an organism whose genomic DNA is desired to be modified and can be a variety of organisms, including the prokaryotes and eukaryotes mentioned above. The organism may be a mature individual, an embryo or fetus in the developmental stage, or in the case of plants, a seed, seedling, or bulb.

In an embodiment, the cell excludes human gametes and fertilized eggs and can be represented as a cell excluding human gametes and fertilized eggs.

In an embodiment, the organism excludes humans and can be represented as a non-human organism. In another embodiment, the organism excludes humans and animals and can be represented as a non-human, non-animal organism.

Further, in an embodiment, the cell excludes human gametes and fertilized eggs, and the organism excludes humans. In another preferred embodiment, the cell excludes human gametes and fertilized eggs, and the organism excludes humans and animals.

Treatment of the cell or organism using the kit for genome editing means making the genome-editing polynucleotide, its expression vector, or a conjugate of the genome-editing polynucleotide or its expression vector with one or more other substances, and the promoting composition for genome editing, coexist with the cell, or administering them to the organism. By this coexistence with the cell or administration to the organism, the genome-editing polynucleotide, its expression vector, or a conjugate of the genome-editing polynucleotide or its expression vector with one or more other substances, and the promoting composition for genome editing, are introduced into the cell or organism, thereby the genomic DNA in the cell or organism can be edited.

In the treatment using the kit for genome editing, there is no restriction on the order of treatment with the genome-editing polynucleotide, its expression vector, or a conjugate of the genome-editing polynucleotide or its expression vector with one or more other substances, and treatment with the promoting composition for genome editing. The two treatments can be performed simultaneously or separately.

In a preferred embodiment, all these two treatments are performed simultaneously. In another preferred embodiment, treatment with the promoting composition for genome editing is performed first, followed by treatment with the genome-editing polynucleotide, its expression vector, or a conjugate of the genome-editing polynucleotide or its expression vector with one or more other substances.

Treatment of the cell or organism using the genome-editing polynucleotide or the kit for genome editing can be performed by various known methods selected by those skilled in the art in consideration of factors such as the type or nature of the cell or organism. Treatment of the cell or organism can include, for example, microinjection, electroporation, DEAE-dextran treatment, transfection (lipofection) using a transfection reagent (jetPEI (PolyPlus-transfection), Lipofectamine, etc.), transfection using a lipid nanoparticle, and the hydrodynamic method.

In an embodiment, the treatment of a human cell is performed in vitro or ex vivo.

In general, genome editing may increase or decrease the characteristic of a cell or organism, such as nutrient requirements, sensitivity or resistance to chemicals, may confer a characteristic not present in the cell or organism before editing, or may cause the cell or organism to lose a characteristic it had before editing. The desired genome-edited cell or organism can be selected using such a change in characteristic as an indicator. For example, if genome editing is expected to increase or confer drug resistance, the genome-edited cell or organism can be selected by culturing cells treated using the kit for genome editing with an appropriate medium containing the drug, or raising organisms treated using the kit for genome editing with an appropriate feed containing the drug. The method of selection and evaluation of the characteristic of the cells or organisms, and the culture or rearing conditions that enable selection of the cells or organisms can be determined as appropriate within the ordinary practicing capability of those skilled in the art.

The method for genome editing and the method for producing a genome-edited cell or organism described above can include, in addition to the step of treating the cell or organism using the kit for genome editing, the step of selecting a cell or organism whose genomic DNA has been edited using a characteristic of the cell or organism that is changed by the genome-editing polynucleotide as an indicator.

While the invention will be described in more detail by the following examples, it should not be considered as being limited to these examples.

### [Examples]

### Example 1: Effects of Hfq and DnaK in eukaryotic cells

### 1) Genome-editing polynucleotide

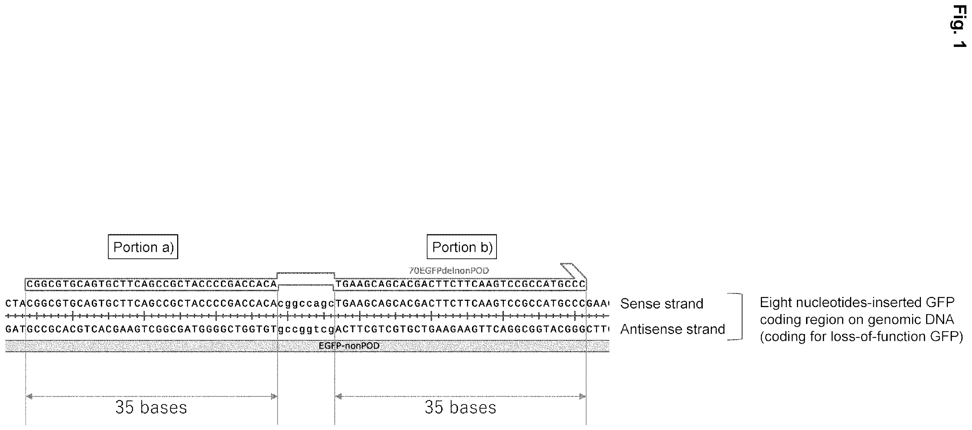

A single-stranded DNA consisting of the nucleotide sequence shown in SEQ ID NO: 17 was synthesized by MacroGen and used as a genome-editing polynucleotide. This genome-editing polynucleotide can delete the inserted nucleotides from the GFP coding sequence (SEQ ID NO: 18) in which 8 nucleotides were inserted to encode loss-of-function GFP. Fig. 1 shows this genome-editing polynucleotide (indicated as 70EGFPdelnonPOD in the figure) aligned with the loss-of-function GFP coding sequence. The 8 nucleotides in the middle of the genomic DNA antisense strand are the primary editing site, and the 8 nucleotides in the middle of the genomic DNA sense strand are the secondary editing site.

### 2) Preparation of cells for assay

HEK293T cells were cultured in Dulbecco's Modified Eagle's Medium-high glucose (DMEM; Sigma, D5796) containing 10% (v/v) Fetal Bovine Serum (Sigma, F7524), 1% (v/v) Antibiotic-Antimycotic (Gibco, 15240-062), at 37° C under 5% CO2 conditions unless otherwise stated.

A cassette containing a loss-of-function GFP gene (SEQ ID NO: 18) was created and introduced into pCDH-CMV-MCS-EF1-RFP-T2A-Puro (System Biosciences, CD516B-2) to construct an expression vector pCDH-CMV-EGFP-nonPOD-1se-polyA-EF1-RFP+puro. This expression vector was transfected into HEK293T cells using Fugene HD (Promega). Transfected HEK293T cells were passaged at 4-6 day intervals in medium containing 0.25 or 0.5 µg/ml of puromycin to drug-select HEK293T cells in which the above cassette was introduced into the genome. The loss-of-function GFP expressed by the cells has a shifted reading frame due to the insertion of 8 nucleotides, resulting in an inactive form of the translated protein, which does not exhibit fluorescence.

### 3) Preparation of Hfq and DnaK

Escherichia coli Hfq (amino acid sequence shown in SEQ ID NO: 2) and E.coli DnaK (amino acid sequence shown in SEQ ID NO: 6) were each prepared as proteins with a nuclear localization signal KKRKK (SEQ ID NO: 10) added to the C-terminus and a His tag and Factor Xa recognition sequence added to the N-terminus, as follows. DNAs encoding E. coli Hfq with the above additional sequences (amino acid sequence shown in SEQ ID NO: 20; nucleotide sequence shown in SEQ ID NO: 19) and E. coli DnaK with the above additional sequences (amino acid sequence shown in SEQ ID NO: 22; nucleotide sequence shown in SEQ ID NO: 21) were prepared, and each was introduced into pET-16b to construct expression vectors pET16b-hfqNLS and pET16b-dnakNLS. These expression vectors were introduced into E. coli strain BL21 (DE3) by electroporation, and Hfq and DnaK proteins were each expressed by culturing at 37°C for 16 hours using Overnight Express(TM) Autoinduction System 1 (Novagen). The bacterial cells were collected, disrupted by sonication, and the soluble fraction obtained by centrifugation was passed through a His Trap HP column (Cytiva), followed by elution of the adsorbed proteins. A 0.7M imidazole solution was used as the elution buffer. Each of the eluted Hfq and DnaK was buffer-exchanged to 20mM-50mM sodium phosphate buffer (pH8.0) containing 0.1 M-0.3M NaCl using a Vivaspin column (Sartorius) or dialysis membrane, and used for genome editing.

### 4) Genome editing by genome-editing polynucleotide

HEK293T cells expressing loss-of-function GFP prepared in step 2) were inoculated into 6-well multi-well plates (Corning, 3516) at 2.5x10⁵ cells/well and incubated in medium containing 10% (v/v) Fetal Bovine Serum (Sigma, F7524), 1% (v /v) Antibiotic-Antimycotic (Gibco, 15240-062) for 2 days to achieve 60-80% confluent cells. Then, 200 µl of DNA-Protein mixture solution (Opti-MEM (Gibco, 11058-021) + Viafect^{™} Transfection Reagent (Promega)) containing 5 µg of genome-editing polynucleotide (70EGFPdelnonPOD) and 0.078-5 µg of Hfq or DnaK was added to each well, and incubated for another 1 day. After removal of the medium, cells were detached and collected using 0.025% Trypsin-EDTA (Gibco, 25300-062), seeded into 10 cm dishes, and cultured in medium containing 1% (v/v) Antibiotic-Antimycotic for 4 days. After removing the medium, cells were detached and collected using 0.025% Trypsin-EDTA.

### 5) Evaluation of genome editing efficiency by flow cytometry

Cells collected in step 4) were resuspended in medium containing 10% (v/v) Fetal Bovine Serum (Sigma, F7524) and 1% (v/v) Antibiotic-Antimycotic, and analyzed by flow cytometry (Attune NxT Flow Cytometer, Thermo Fisher Scientific) to count green fluorescent cells, and the percentage of green fluorescent cells to total cells was calculated as nucleotide deletion rate. The green fluorescent cells were those expressing normal GFP protein, meaning that the 8 nucleotides inserted in the nucleotide sequence encoding the loss-of-function type GFP were deleted by genome editing in those cells.

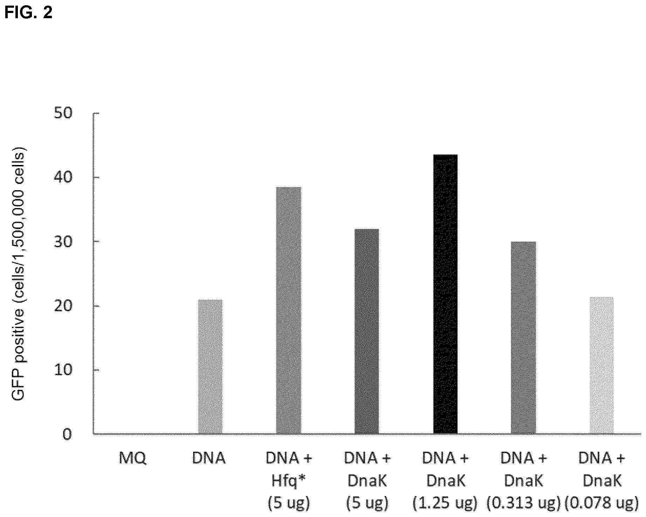

The nucleotide deletion rates by the genome-editing polynucleotide in the presence of Hfq or DnaK are shown in Fig. 2. In the figure, MQ corresponds to cells to which ultrapure water was added instead of DNA-Protein mixture solution, and the numbers in parentheses indicate the amount of Hfq or DnaK used. When the genome-editing polynucleotide was combined with Hfq or DnaK, an enhancement in the nucleotide deletion rate of loss-of-function GFP in HEK293T cells was confirmed compared to the use of genome-editing polynucleotide alone (indicated as DNA in the figure).

### Test Example: Effects of Hfq and DnaK in prokaryotic cells

### 1) Preparation of cells for assay

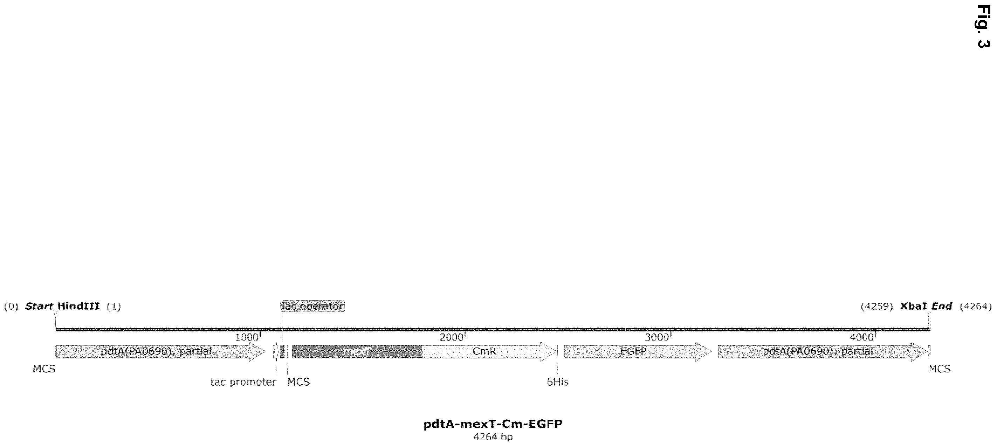

A cassette linking the mexT gene of Pseudomonas aeruginosa, chloramphenicol resistance gene (CmR), and EGFP gene (nucleotide sequence shown in SEQ ID NO: 23 and configuration shown in Fig. 3) was constructed and introduced downstream of the tac promoter of pMMB67EH to obtain an assay plasmid. The assay plasmid was introduced into wild-type P. aeruginosa strain PAO1S-Lac to create PAO1S-Lac-mexTCmEGFP cells for assay.

The mexT gene in this assay plasmid does not express active MexT protein that would be translated in the correct frame, nor does it express the downstream CmR and EGFP genes. The mexT gene has a structure where sequence X-Y-X overlaps at X (X-Y-X-Y-X): CGGCCA-GC-CGGCCA-GC-CGGCCA (SEQ ID NO: 24). It is known that nucleotide sequences with such a structure often delete the non-overlapping sequence X-Y (see, for example, Japanese Patent Application Publication No. 2016-220639), and in the case of the mexT gene, deletion of the non-overlapping 8 nucleotides (CGGCCA-GC) occurs. The occurrence of deletion can be confirmed by the expression of the downstream CmR and EGFP genes as a result of the frameshift caused by the deletion, which can be observed as chloramphenicol resistance and EGFP fluorescence.

### 2) Construction of expression vectors for Hfq and DnaK

The coding DNA for P. aeruginosa Hfq (amino acid sequence shown in SEQ ID NO: 4; nucleotide sequence shown in SEQ ID NO: 3), the coding DNA for P. aeruginosa DnaK (amino acid sequence shown in SEQ ID NO: 8; nucleotide sequence shown in SEQ ID NO: 7), or both of these DNAs were introduced into pMMB67EH by In-Fusion^{R} (Clontech) to construct expression vectors pMMB67EH-hfq, pMMB67EH-dnaK, and pMMB67EH-dnaK-hfq.

### 3) Introduction of Hfq and DnaK expression vectors and evaluation of nucleotide deletion rates

After introducing each of the expression vectors from 2) or empty vector (pMMB67EH) into the assay cells, single colonies were isolated by plating on LB plates containing 150 µg/ml chloramphenicol. After pre-culturing in LB medium containing 150 µg/ml chloramphenicol, the cells were subcultured into a 10-fold volume of LB medium containing 1 mM IPTG and cultured at 37°C for 4.5 hours until OD600 reached approximately 0.7, after which the bacterial cells were collected from the culture. After washing the cells, green fluorescent cells were counted using a flow cytometer (Attune NxT Flow Cytometer, Thermo Fisher Scientific), and the nucleotide deletion rate was calculated as the percentage of green fluorescent cells among all cells.

The results are shown in Fig. 4. In the figure, "none" indicates cells introduced with empty vector (pMMB67EH), "dnaK" indicates cells introduced with pMMB67EH-dnaK, "dnaK-hfq" indicates cells introduced with pMMB67EH-dnaK-hfq, and "hfq" indicates cells introduced with pMMB67EH-hfq. It was confirmed that Hfq and DnaK each independently enhanced the nucleotide deletion rate of the mexT gene in P. aeruginosa, and that the combination of Hfq and DnaK further enhanced the nucleotide deletion rate compared to their individual use.

## Claims

1. A composition for increasing genome-editing efficiency by a single-stranded genome-editing polynucleotide capable of modifying a target site on a double-stranded genomic DNA, containing at least one protein selected from the group consisting of Hfq, DnaK, an Hfq variant consisting of an amino acid sequence having 90% or more identity with the amino acid sequence of Hfq and having an activity of increasing genome-editing efficiency, and a DnaK variant consisting of an amino acid sequence having 90% or more identity with the amino acid sequence of DnaK and having an activity of increasing genome-editing efficiency, or a nucleic acid containing a nucleotide sequence encoding the protein,
wherein the target site consists of a primary editing site set on one genomic DNA strand and a secondary editing site on the other genomic DNA strand at a position corresponding to the primary editing site,
the genome-editing polynucleotide consists of, in order from its 5' terminal side,
a) a portion capable of hybridizing to the region adjacent to the 3' terminal side of the primary editing site,
x) an optional portion consisting of a nucleotide sequence that is not identical to the nucleotide sequence of the secondary editing site, and
b) a portion capable of hybridizing to the region adjacent to the 5' terminal side of the primary editing site, or
the genome-editing polynucleotide consists of, in order from its 5' terminal side, portion a) and portion b), and
when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
when the genome-editing polynucleotide consists of portion a) and portion b), and the primary editing site is set at one nucleotide or multiple contiguous nucleotides on the one genomic DNA strand, the genome-editing polynucleotide can delete the target site.

2. The composition according to claim 1, wherein Hfq consists of the amino acid sequence shown in SEQ ID NO: 2 or 4.

3. The composition according to claim 1, wherein DnaK consists of the amino acid sequence shown in SEQ ID NO: 6 or 8.

4. The composition according to any one of claims 1 to 3, wherein the protein has a nuclear localization signal added thereto.

5. The composition according to any one of claims 1 to 3, wherein the protein has a nuclear localization signal consisting of the amino acid sequence shown in any one of SEQ ID NOs: 9 to 16 added thereto.

6. A kit for genome editing, containing:
the composition according to claim 1, and
a single-stranded genome-editing polynucleotide, an expression vector thereof, or a conjugate of the genome-editing polynucleotide or the expression vector thereof with one or more other substances,
wherein the genome-editing polynucleotide consists of, in order from its 5' terminal side,
a) a portion capable of hybridizing to the region adjacent to the 3' terminal side of the primary editing site,
x) an optional portion consisting of a nucleotide sequence that is not identical to the nucleotide sequence of the secondary editing site, and
b) a portion capable of hybridizing to the region adjacent to the 5' terminal side of the primary editing site, or
the genome-editing polynucleotide consists of, in order from its 5' terminal side, portion a) and portion b),
the lengths of portions a) and b) are each independently 10 to 100 nucleotides, and
when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
when the genome-editing polynucleotide consists of portion a) and portion b), and the primary editing site is set at one nucleotide or multiple contiguous nucleotides on the one genomic DNA strand, the genome-editing polynucleotide can delete the target site.

7. The kit for genome editing according to claim 6, containing the single-stranded genome-editing polynucleotide containing DNA high-affinity nucleotide analogs as constituent units, or a conjugate of the genome-editing polynucleotide with one or more other substances.

8. A method for modifying a target site on a double-stranded genomic DNA of a cell excluding human gametes and fertilized eggs, or a non-human organism, comprising the step of treating the cell or the organism using the kit for genome editing according to claim 6 or 7, provided that when the cell is a human cell excluding human gametes and fertilized eggs, the step is performed in vitro or ex vivo.

9. A method for producing a cell excluding human gametes and fertilized eggs, or a non-human organism in which a target site on a double-stranded genomic DNA has been modified, comprising the step of treating the cell or the organism using the kit for genome editing according to claim 6 or 7, provided that when the cell is a human cell excluding human gametes and fertilized eggs, the step is performed in vitro or ex vivo.
